# EUROPEAN PATENT SPECIFICATION

(11) **EP 0 497 206 B1**
(45) Date of publication and mention of the grant of the patent: **07.01.1998**
(21) Application number: 92101037.7
(22) Date of filing: 23.01.1992
(51) Int. Cl.: C07C 2/14, C07C 2/20

(54) **Process for producing olefin oligomer**
Verfahren zur Herstellung von Olefinoligomer
Procédé pour la préparation d'oligomère d'oléfine

(30) Priority: 01.02.1991 JP 12174/91
(43) Date of publication of application: 05.08.1992
(73) Proprietor: IDEMITSU PETROCHEMICAL CO., LTD., Minato-ku, Tokyo (JP)
(72) Inventor: Akatsu, Makoto, c/o Idemitsu Petrochemical, Tokuyama-shi, Yamaguchi-ken (JP); Kawamura, Tatsuya, c/o Idemitsu Petrochemical, Tokuyama-shi, Yamaguchi-ken (JP); Kutsuno, Takayoshi, c/o Idemitsu Petrochemical, Tokuyama-shi, Yamaguchi-ken (JP)
(74) Representative: Hrabal, Ulrich, Dr.

(56) References cited:
- EP-A- 0 349 278
- EP-A- 0 352 723
- GB-A- 2 024 846
- US-A- 4 239 930
- US-A- 4 308 414
- US-A- 4 365 105
- US-A- 4 479 023

## Description

### BACKGROUND OF THE INVENTION

### Field of the Invention

The present invention relates to a process for producing an olefin oligomer.

### Prior Art

It is known to produce an olefin oligomer by polymerizing an olefin, especially an α-olefin having 6 to 12 carbon atoms. The olefin oligomer is useful as a base fluid for an automotive engine oil, an aviation hydraulic fluid, an electrical insulating oil, and the like. In particular, the usefulness of an olefin oligomer having a comparatively low viscosity as a base fluid for an automotive engine oil has been increasing.

For producing an olefin oligomer, there is known a cationic polymerization method using, as a catalyst, a Lewis acid such as boron trifluoride.

In the above method using boron trifluoride as a catalyst, an olefin oligomer having a relatively low viscosity can be produced. However, there is the defect in this method that it is difficult to control the polymerization reaction rate. In particular, since the initial rate of polymerization is very fast, it is required to provide the polymerization reactor with an internal cooling coil or an external heat exchanger to remove heat of the reaction. Further, since the polymerization rate decreases as the polymerization proceeds, it is required to control the feed rate of a refrigerant to the internal cooling coil or to the external heat exchanger in order to keep a constant temperature in the polymerization reactor.

On the other hand, in a process for the production of an olefin oligomer, boron trifluoride is usually used in combination with a cocatalyst such as water, an alcohol or a carboxylic acid which forms a complex with the boron trifluoride. And in this case, it is known that the amount of boron trifluoride is required to be larger than the amount that is necessary to form a complex with the above cocatalyst. In the conventional process, boron trifluoride is fed to the reactor which is maintained at a pressure higher than atmospheric pressure with boron trifluoride, or boron trifluoride under atmospheric pressure is blown into a reaction liquid. In the other conventional process, an olefin saturated with boron trifluoride at a pressure equivalent to, or higher than, atmospheric pressure and a complex of boron trifluoride with a cocatalyst are separately fed to the reactor. In the above conventional catalyst feed methods, therefore, an olefin is always saturated with boron trifluoride having a pressure equivalent to, or higher than, atmospheric pressure. However, when an olefin is saturated with boron trifluoride as described above, there arises the above disadvantage that the controlling of the polymerization reaction rate is difficult.

GB-A-2 024 846 discloses a synthetic hydrocarbon product particularly suitable as a constituent of crankcase lubricant oils for internal combustion engines which is made by oligomerizing one or more C₁₂₋₁₈ alpha olefins. The oligomerization is carried out in the presence of a Friedel-Crafts catalyst (boron trifluoride, promoted with water, being especially preferred) and at 20 to 200°C.

EP-A-0 352 723 discloses the oligomerization of C₈₋₁₄ α-olefins to a oligomer mixture containing a high content of tetramer by feeding the α-olefin to a reaction vessel at a controlled rate over an extended period and in the presence of BF₃ and a promoter such as an alcohol.

EP-A-0 349 278 discloses the making of synlubes having a consistently low pour point by feeding a promoter (e.g. water, alcohol) at a controlled rate over an extended time period to an α-olefin in contact with boron trifluoride.

US 4,239,930 discloses the oligomerization of alpha-olefins to a product useful as synthetic lubricant in a continuous process utilizing a promoted boron trifluoride catalyst. The oligomerization is carried cut in a system comprising a plurality of loop recycle reactors arranged in series.

US 4,365,105 discloses the oligomerization of an alpha-olefin in the presence of a three-component catalyst comprising a particulate solid adsorbent, boron trifluoride and elemental oxygen.

US 4,308,414 discloses the oligomerization of an alpha-olefin in the presence of a three-component catalyst comprising a particulate solid adsorbent having boron trifluoride and water adsorbed on the solid adsorbent.

US 4,479,023 discloses a process for the oligomerization of olefin fractions where boron trifluoride in the gas phase is contacted with such a fraction, in the absence of a catalyst carrier, under such conditions and for such a length of time that the isobutene and/or butadiene present will polymerize; the so obtained isobutene and/or butadiene polymers are conventionally separated and oligomerized in the presence of a boron trifluoride catalyst comprising a catalyst carrier.

### SUMMARY OF THE INVENTION

It is therefore an object of the present invention to provide a process for producing an olefin oligomer, in which the olefin polymerization reaction rate can be controlled.

The present inventors have made a study to achieve the above object, and found the following: At least, at an initial time of the polymerization, the concentration of boron trifluoride is kept lower than the saturated concentration of boron trifluoride in an olefin at atmospheric pressure, whereby the polymerization reaction rate can be controlled. The present invention has been completed on the basis of the above finding.

Therefore, the gist of the present invention consists in a process for producing an olefin oligomer which comprises polymerizing an olefin having 4 to 18 carbon atoms in the presence of boron trifluoride and a cocatalyst selected from the group consisting of water, alcohols, carboxylic acids, ethers, acid anhydrides, esters, ketones and aldehydes at a temperature of from -20 to 90°C characterized in that at least at an initial time of the polymerization the partial pressure of the boron trifluoride in the reactor is adjusted to less than 0,0981 MPa (1 kg/cm²) by using a mixed gas containing boron trifluoride.

### BRIEF EXPLANATION OF THE DRAWING

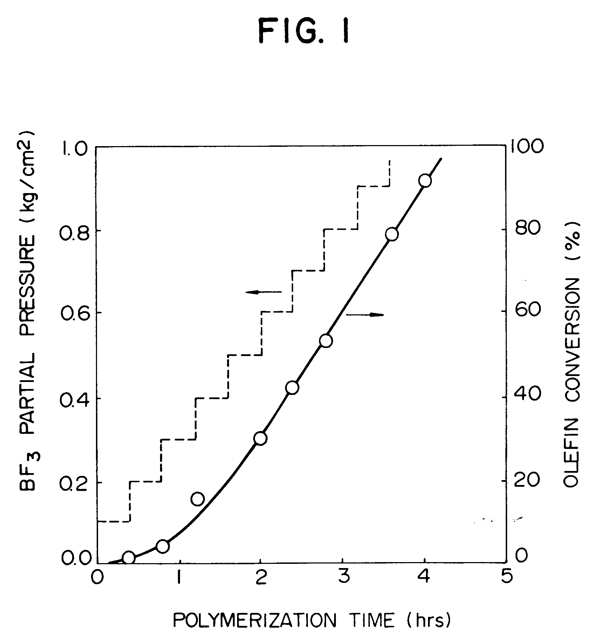

Figure 1 shows changes of the BF₃ partial pressure and the olefin conversion with time in Example 5.

### DETAILED DESCRIPTION OF THE INVENTION

The present invention will be detailed hereinafter.

The olefin used as a raw material in the process for producing an olefin oligomer, provided by the present invention, is an α-olefin having 8 to 14 carbon atoms.

The above olefin as a raw material is polymerized in the presence of boron trifluoride and its cocatalyst. The specific meaning of the phrase "in the presence of boron trifluoride and its cocatalyst" is that a boron trifluoride-cocatalyst complex obtained from boron trifluoride and its cocatalyst is present together with boron trifluoride. Therefore, the cocatalyst includes any compounds which react with boron trifluoride to form a complex having polymerization activity, and mixtures of these compounds. Specific examples of the cocatalyst are water; alcohols such as methanol, ethanol, n-butanol and n-decanol; carboxylic acids such as acetic acid, propionic acid and butyric acid; ethers such as dimethyl ether and diethyl ether; anhydrides such as acetic anhydride and succinic anhydride; esters such as ethyl acetate and methyl propionate; ketones such as acetone and methyl ethyl ketone; and aldehydes such as acetaldehyde and benzaldehyde.

Although not specially limited, the amount of the above complex based on the olefin is generally 0.05 to 1 % by weight, preferably 0.1 to 5 % by weight.

The process for producing an olefin oligomer, provided by the present invention, is characterized in that, at least at an initial time of the polymerization, the concentration of boron trifluoride in an olefin is kept lower than the saturated concentration of boron trifluoride at atmospheric pressure. Due to this, the polymerization rate, particularly the polymerization rate at an initial time of the polymerization, can be controlled. As a result, the following advantages are achieved: (i) The reaction heat per unit time can be so controlled to be almost constant, and the temperature in the reactor is easily controlled. (ii) An olefin oligomer can be produced in a simple apparatus.

As described above, at least at an initial time of the polymerization, the concentration of boron trifluoride in the olefin is kept lower than the saturated concentration of boron trifluoride in the olefin at atmospheric pressure. The concentration of boron trifluoride may be kept lower than a saturated concentration thereof from the beginning of the polymerization to the end of the polymerization. When a predetermined period of time has passed after the start of the polymerization, the concentration of boron trifluoride may be kept equivalent to, or higher than, the saturated concentration.

The concentration of boron trifluoride can be kept lower by controlling the feed amount of boron trifluoride or the partial pressure of boron trifluoride in the reactor. The saturated concentration in an olefin at atmospheric pressure varies depending upon the kind of the olefin, the temperature in the polymerization system, and the like. When the olefin is 1-decene and when the temperature in the polymerization system is 20° C, the saturated concentration of boron trifluoride is about 0.21 g/100 g of the olefin. Therefore, the feed amount of boron trifluoride is controlled such that the concentration thereof is lower than the above saturated concentration, or the partial pressure of boron trifluoride in the reactor is adjusted such that it is less than 0.0981 MPa (1 kg/cm²), whereby the concentration of boron trifluoride in the olefin is kept lower than its saturated concentration at atmospheric pressure.

The concentration of boron trifluoride during the polymerization may be kept at a substantially constant level, or may be changed continuously or stepwise.

No solvent is particularly required. However, an organic solvent may be used as required. The organic solvent is selected from halogenated hydrocarbons such as carbon tetrachloride, chloroform and methylene chloride; aliphatic saturated hydrocarbons such as pentane, hexane and heptane; and alicyclic hydrocarbons such as cyclohexane, methylcyclohexane and decalin.

The polymerization temperature is -20 to 90° C, preferably -10 to 60° C.

The present invention will be described further in detail by reference to Examples, although it shall not be limited thereto.

### Example 1

(1) A polymerization flask equipped with a stirrer, a thermometer, a gas introducing tube and a gas exhausting tube was flushed with dry nitrogen gas, and charged with 200 ml of 1-decene and a boron trifluoride·n-butanol complex in an amount that was equivalent to 1.68 mol per 100 mol of 1-decene. The resultant mixture was cooled to 20° C, and a boron trifluoride/nitrogen mixed gas of which the boron trifluoride partial pressure had been adjusted to 0.0491 MPa (0.5 kg/cm²) was blown into the mixture to initiate the polymerization. When the polymerization was initiated, the concentration of boron trifluoride in the olefin was about 0.1 % by weight, which was lower than its saturated concentration at atmospheric pressure.
   After the start of the polymerization, the polymerization was continued for 1 hour while the temperature was kept at 20° C by cooling the reaction mixture. During the polymerization for 1 hour, no sharp increase was observed in the polymerization reaction rate, and the polymerization reaction rate was very easily controlled.
   After the above one-hour polymerization, a portion of the reaction mixture was sampled, and 5 % ammonia water was added to deactivate the catalyst. Then, the sampled reaction mixture was washed with water and dried, and an unreacted olefin and a low molecular weight oligomer having 20 or less carbon atoms were distilled off to give an olefin oligomer. Table 1 shows the olefin conversion and the composition, yield and properties of the oligomer. The composition of the oligomer shows the analysis value before the low molecular weight oligomer was distilled off. The analysis values except for the properties were determined by gas chromatography.
   As shown in Table 1, the olefin conversion was 52.8 % and the yield of the oligomer was 50.4 %. These results also show that the polymerization was carried out with the polymerization reaction rate under control.
(2) The rest of the above reaction mixture was further subjected to polymerization for 7 hours (total polymerization time; 8 hours). During this polymerization for 7 hours, no sharp increase was observed in the polymerization reaction rate, and the polymerization reaction rate was very easily controlled.
   After the polymerization, the reaction mixture was treated in the same manner as in (1) to give an olefin oligomer. Table 1 shows the olefin conversion, and the composition, yield and properties of the oligomer.
   As shown in Table 1, the olefin conversion was 99.9 %, and the yield of the oligomer was 98.8 %. These results also show that the polymerization for the second period of 7 hours was carried out with the polymerization rate under control.
   The results in the above (1) and (2) show that, according to Example 1, the polymerization can be carried out with the polymerization reaction rate under control from the beginning of the polymerization to the end of the polymerization, particularly at the initial time of the polymerization.

### Comparative Example 1

(1) Example 1(1) was repeated except that the boron trifluoride/nitrogen mixed gas was replaced with a boron trifluoride gas and that the partial pressure of boron trifluoride was changed to 0.0981 MPa (1.0 kg/cm²) (corresponding to a case in which the olefin was saturated with boron trifluoride). In the one-hour polymerization, the polymerization reaction rate increased sharply, and it was very difficult to control the polymerization reaction rate.
   As shown in Table 1, the olefin conversion was 87.3 %, and the yield of the oligomer was 84.9 %. These results also show that it was very difficult to control the polymerization reaction rate.
(2) The above reaction mixture was further subjected to polymerization (total polymerization time; 2 hours). As a result, the olefin conversion was 98.6 % and the yield of the olefin was 97.3 % as shown in Table 1. These results show that the polymerization for the second period of 1 hour proceeded very slowly, differing from the polymerization in (1).
   The results in the above (1) and (2) show the following; According to Comparative Example 1, the initial rate of polymerization is very fast, and the polymerization reaction rate extraordinarily decreases after the lapse of 1 hour. It is therefore difficult to control the polymerization reaction rate.

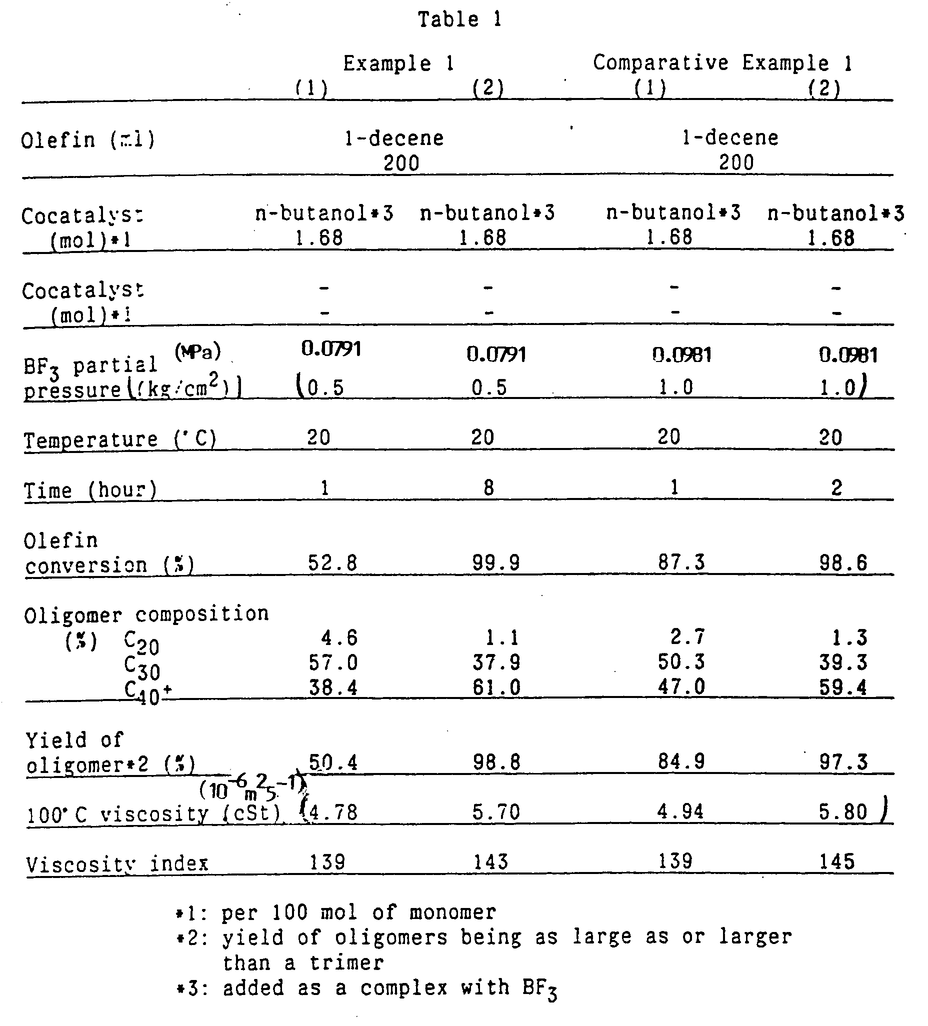

### Examples 2 and 3

1-Decene was polymerized in the same manner as in Example 1 except that the partial pressure of boron trifluoride was adjusted to 0.1962 MPa (0.2 kg/cm²) (less than the saturated concentration at atmospheric pressure) in Example 2 or that the partial pressure of boron trifluoride was adjusted to 0.00981 MPa (0.1 kg/cm²) (less than the saturated concentration at atmospheric pressure) in Example 3 as shown in Table 2. The results in these two Examples show that the reaction rate at an initial time of the polymerization can be easily controlled. This is also clear from changes of the olefin conversions and yields of oligomers with time, shown in Table 2.

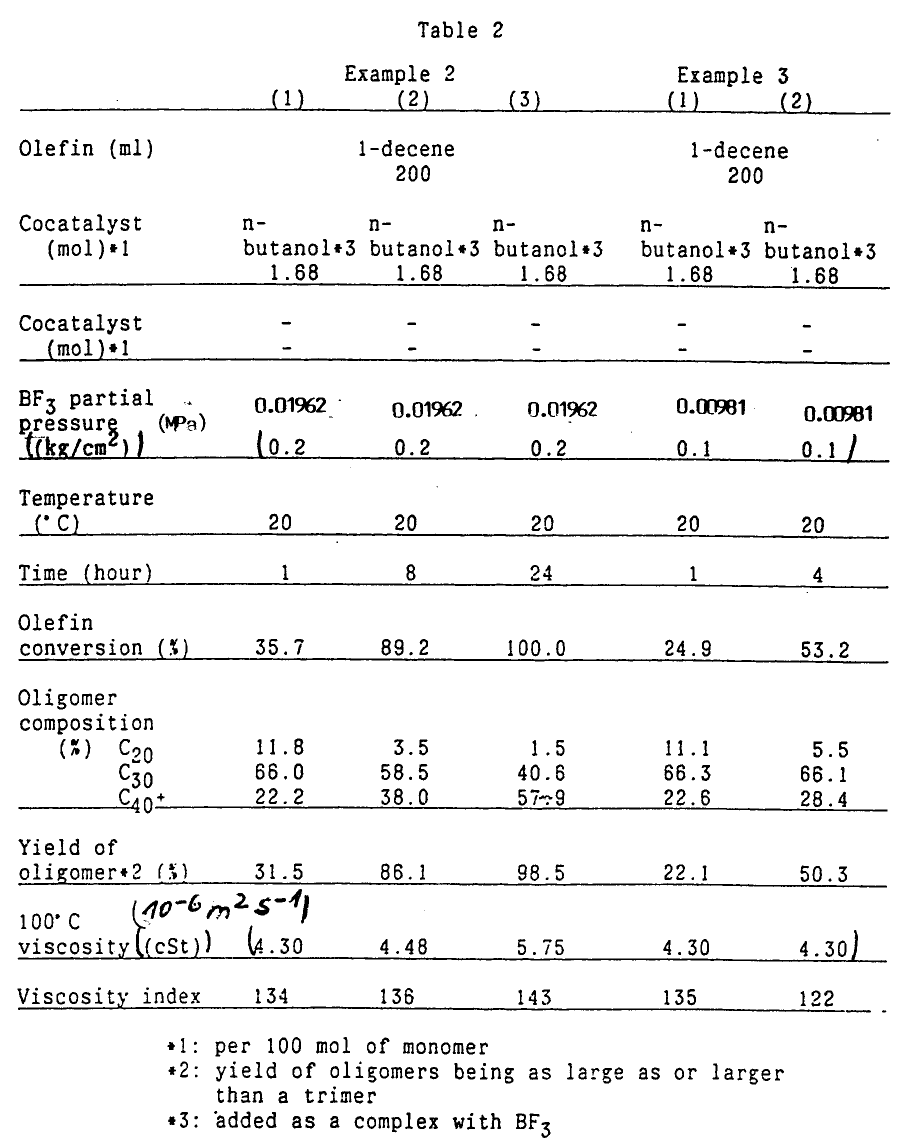

### Example 4 and Comparative Example 2

In each of Example 4 and Comparative Example 2, 1-decene was polymerized under the same conditions shown in Table 3 except that the partial pressure of boron trifluoride in Example 4 was adjusted to 0.01962 MPa (0.2 kg/cm²) (less than the saturated concentration at atmospheric pressure) and that the partial pressure of boron trifluoride in Comparative Example 2 was adjusted to 0.0981 MPa (1.0 kg/cm²) (saturated concentration at atmospheric pressure).

The results in these two Examples show that the polymerization reaction rate can be more easily controlled in Example 4 than in Comparative Example 2. This is also clear from the results of olefin conversions and yields of oligomers shown in Table 3.

### Example 5

1-Decene was polymerized in the same manner as in Example 4 except that, as shown in Figure 1, the partial pressure of boron trifluoride was increased stepwise from 0.00981 MPa (0.1 kg/cm²) (less than the saturated concentration at atmospheric pressure) to 0.0981 MPa (1.0 kg/cm²) (saturated concentration at atmospheric pressure) and that the polymerization time was changed to 4 hours.

As shown in Figure 1, the results show that the olefin conversion increases parallel with an increase in the partial pressure of boron trifluoride, and that the polymerization reaction rate can be therefore very easily controlled.

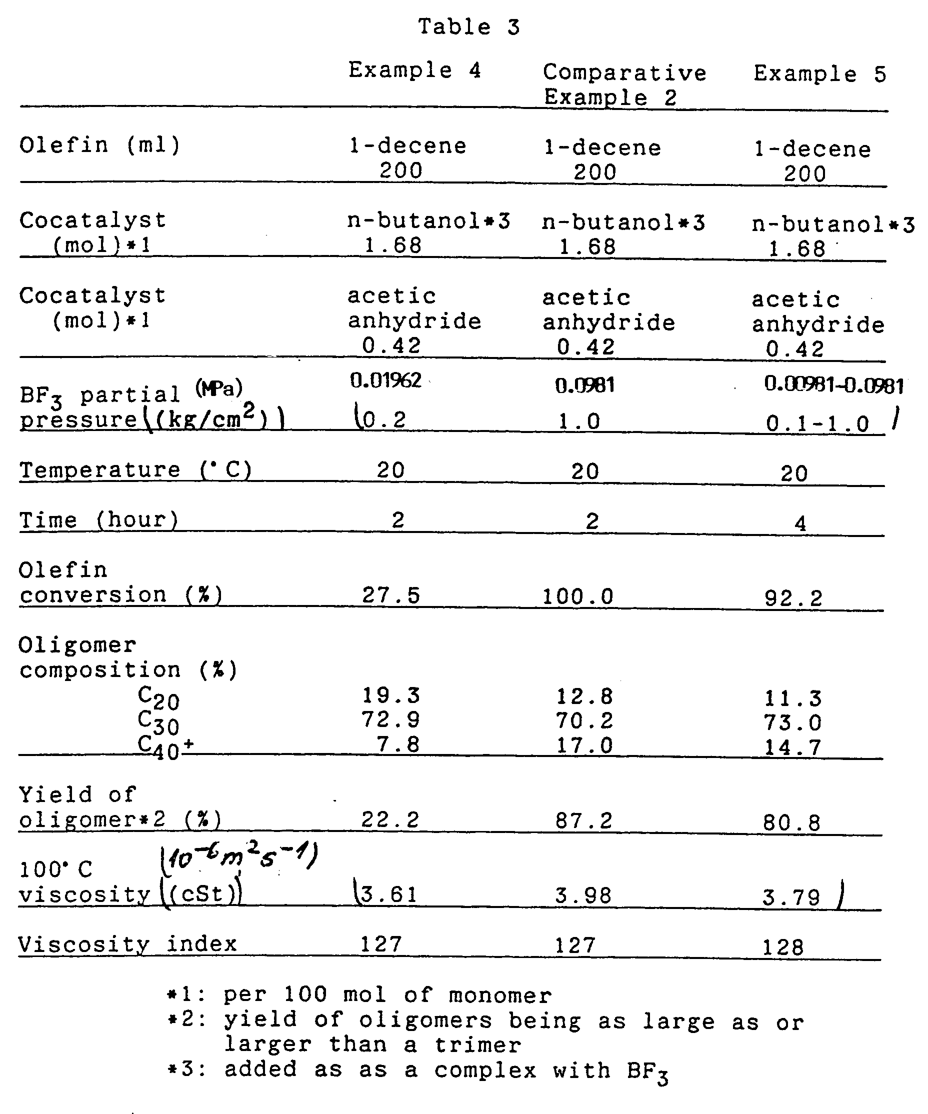

### Example 6 and Comparative Example 3

In each of Example 6 and Comparative Example 3, 1-octene was polymerized under the same conditions shown in Table 4 except that the partial pressure of boron trifluoride in Example 6 was adjusted to 0.01962 MPa (0.2 kg/cm²) (less than the saturated concentration at atmospheric pressure) and that the partial pressure of boron trifluoride in Comparative Example 3 was adjusted to 0.0981 MPa (1.0 kg/cm²) (saturated concentration at atmospheric pressure).

The results in these two Examples show that the polymerization reaction rate can be more easily controlled in Example 6 than in Comparative Example 3. This is also clear from the results of the olefin conversions and yields of oligomers shown in Table 4.

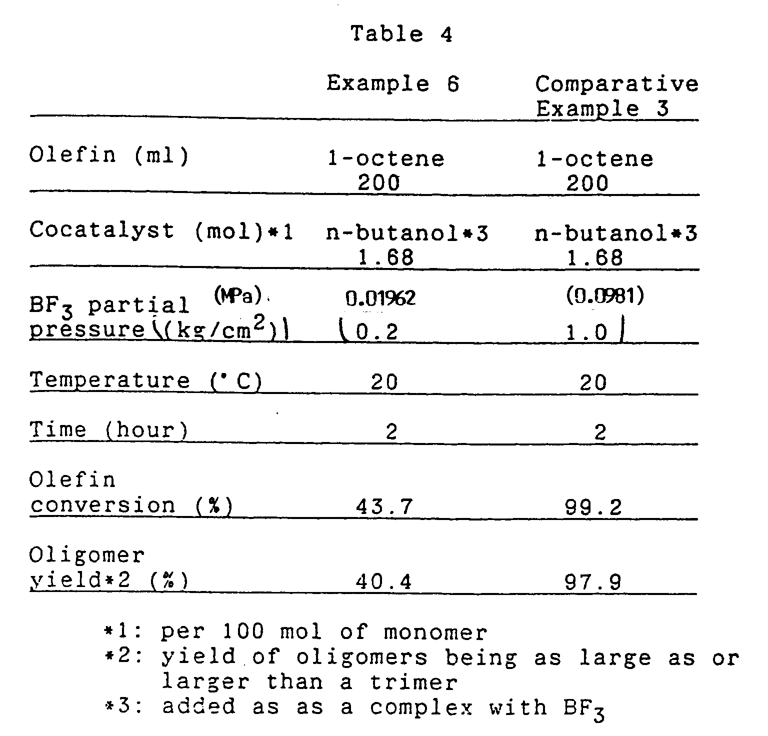

### Example 7 and Comparative Example 4

In each of Example 7 and Comparative Example 4, 1-dodecene was polymerized under the same conditions shown in Table 5 except that the partial pressure of boron trifluoride in Example 7 was adjusted to 0.01962 MPa (0.2 kg/cm²) (less than the saturated concentration at atmospheric pressure) and that the partial pressure of boron trifluoride in Comparative Example 4 was adjusted to 0.0981 MPa (1.0 kg/cm²) (saturated concentration at atmospheric pressure).

The results in these two Examples show that the polymerization reaction rate can be more easily controlled in Example 7 than in Comparative Example 4. This is also clear from the results of olefin conversions and yields of oligomers shown in Table 5.

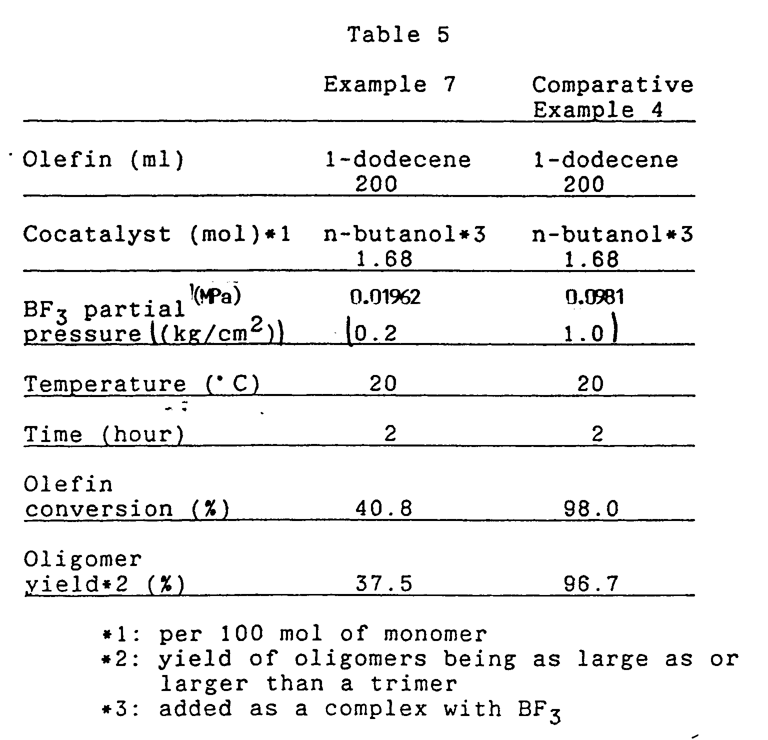

As specified above, the present invention provides a process for producing an olefin oligomer in which the olefin polymerization rate, particularly that at an initial time of the polymerization, can be controlled.

## Claims

1. A process for producing an olefin oligomer which comprises polymerizing an olefin having 4 to 18 carbon atoms in the presence of boron trifluoride and a cocatalyst selected from the group consisting of water, alcohols, carboxylic acids, ethers, acid anhydrides, esters, ketones and aldehydes at a temperature of from -20 to 90°C characterized in that at least at an initial time of the polymerization the partial pressure of the boron trifluoride in the reactor is adjusted to less than 0,0981 MPa (1 kg/cm²) by using a mixed gas containing boron trifluoride.

2. A process according to claim 1, wherein the partial pressure in the reactor is adjusted to 0,00981 to 0,0491 MPa (0.1 to 0.5 kg/cm²).

3. A process according to claim 1, wherein the partial pressure of the boron trifluoride is kept lower than 0,0981 MPa (1 kg/cm²) from the beginning of the polymerization to the end of the polymerization.

4. A process according to claim 1, wherein the partial pressure of the boron trifluoride in the reactor is kept lower than 0,0981 MPa (1 kg/cm²) and, when a predetermined period of time has passed after the start of the polymerization, the concentration of the boron trifluoride is kept equal to or higher than 0,0981 MPa (1 kg/cm²).

5. A process according to claim 1, wherein the olefin is 1-decene.

6. A process according to claim 1, wherein the partial pressure of the boron trifluoride in the reactor during the polymerization is maintained at a substantially constant level.

7. A process according to claim 1, wherein the partial pressure of the boron trifluoride in the reactor during the polymerization is continuously or stepwise changed.

8. A process according to claim 1, wherein the olefin is an α-olefin having 6 to 18 carbon atoms.

9. A process according to claim 1, wherein the complex formed by the reaction of the cocatalyst with boron trifluoride is present in an amount of 0.05 to 10 percent by weight based on the olefin.

10. A process according to claim 1, wherein the polymerization is carried out in an organic solvent.

## Patentansprüche

1. Verfahren zur Herstellung eines Olefin-Oligomeren, welches Polymerisieren eines Olefins mit 4 bis 18 Kohlenstoffatomen bei einer Temperatur von -20 bis 90°C in Gegenwart von Bortrifluorid und einem Cokatalysator, der ausgewählt wird aus der aus Wasser, Alkoholen, Carbonsäuren, Ethern, Säureanhydriden, Estern, Ketonen und Aldehyden bestehenden Gruppe umfaßt, dadurch charakterisiert, daß mindestens zur Anfangszeit der Polymerisation der Partialdruck des Bortrifluorids im Reaktor auf weniger als 0,0981 MPa (1 kg/cm²) eingestellt wird, indem ein Bortrifluorid enthaltendes gemischtes Gas verwendet wird.

2. Verfahren nach Anspruch 1, wobei der Partialdruck im Reaktor auf 0,00981 bis 0,0491 MPa (0,1 bis 0,5 kg/cm²) eingestellt wird.

3. Verfahren nach Anspruch 1, wobei der Partialdruck des Bortrifluorids vom Anfang der Polymerisation bis zum Ende der Polymerisation geringer gehalten wird als 0,0981 MPa (1 kg/cm²).

4. Verfahren nach Anspruch 1, wobei der Partialdruck des Bortrifluorids im Reaktor geringer gehalten wird als 0,0981 MPa (1 kg/cm²) und nach Ablauf einer vorherbestimmten Zeit nach dem Start der Polymerisation die Konzentration des Bortrifluorids gleich oder höher gehalten wird als 0,0981 MPa (1 kg/cm²).

5. Verfahren nach Anspruch 1, wobei das Olefin 1-Decen ist.

6. Verfahren nach Anspruch 1, wobei der Partialdruck des Bortrifluorids im Reaktor während der Polymerisation auf einem im wesentlichen konstanten Wert gehalten wird.

7. Verfahren nach Anspruch 1, wobei der Partialdruck des Bortrifluorids im Reaktor während der Polymerisation kontinuierlich oder schrittweise geändert wird.

8. Verfahren nach Anspruch 1, wobei das Olefin ein a-Olefin mit 6 bis 18 Kohlenstoffatomen ist.

9. Verfahren nach Anspruch 1, wobei der durch Reaktion des Cokatalysators mit Bortrifluorid gebildete Komplex in einer Menge von 0,05 bis 10 Gew.-%, bezogen auf das Olefin, anwesend ist.

10. Verfahren nach Anspruch 1, wobei die Polymerisation in einem organischen Lösungsmittel durchgeführt wird.

## Revendications

1. Un procédé pour la préparation d'un oligomère d'oléfine qui comporte la polymérisation d'une oléfine ayant de 4 à 18 atomes de carbone en présence de trifluorure de bore et d'un cocatalyseur choisi dans le groupe constitué par l'eau, les alcools, les acides carboxyliques, les éthers, les anhydrides d'acide, les esters, les cétones et les aldéhydes à une température de -20 à 90°C, caractérisé en ce qu'au moins au moment initial de la polymérisation, la pression partielle de trifluorure de bore dans le réacteur est ajustée à moins de 0,0981 MPa (1 kg/cm²) en utilisant un gaz mixte renfermant du trifluorure de bore.

2. Un procédé selon la revendication 1, dans lequel la pression partielle dans le réacteur est réglée entre 0,00981 et 0,0491 MPa (0,1 à 0,5 kg/cm²).

3. Un procédé selon la revendication 1, dans lequel la pression partielle de trifluorure de bore est maintenue inférieure à 0,0981 MPa (1 kg/cm₂) à partir du début de la polymérisation jusqu'à la fin de cette dernière.

4. Un procédé selon la revendication 1, dans lequel la pression partielle de trifluorure de bore dans le réacteur est maintenue inférieure à 0,0981 MPa (1 kg/cm²) et, lorsqu'une période prédéterminée de temps s'écoule après le début de la polymérisation, la concentration en trifluorure de bore est maintenue égale ou supérieure à 0,0981 MPa (1 kg/cm²).

5. Un procédé selon la revendication 1, dans lequel l'oléfine est du 1-décène.

6. Un procédé selon la revendication 1, dans lequel la pression partielle de trifluorure de bore dans le réacteur pendant la polymérisation est maintenue à un niveau essentiellement constant.

7. Un procédé selon la revendication 1, dans lequel la pression partielle du trifluorure de bore dans le réacteur pendant la polymérisation est modifiée en continu ou par étapes.

8. Un procédé selon la revendication 1, dans lequel l'oléfine est une α-oléfine qui présente de 6 à 18 atomes de carbone.

9. Un procédé selon la revendication 1, dans lequel le complexe formé par la réaction entre le cocatalyseur et le trifluorure de bore est présent en une quantité de 0,05 à 10% en poids en se basant sur l'oléfine.

10. Un procédé selon la revendication 1, dans lequel la polymérisation est mise en oeuvre dans un solvant organique.
